# EUROPEAN PATENT APPLICATION

(11) **EP 2 292 165 A2**
(43) Date of publication of application: **09.03.2011**
(21) Application number: 10251508.7
(22) Date of filing: 27.08.2010
(51) Int. Cl.: A61B 17/34

(54) **A foam port introducer**

(30) Priority: 31.08.2009 US 238228 P; 30.07.2010 US 847022
(71) Applicant: Tyco Healthcare Group, LP, New Haven CT 06511 (US)
(72) Inventor: O'Keefe, Jonathan B., North Attleboro, MA 02760 (US); Ransden, Jeffrey E., Fairfield, CT 06825 (US); Stellon, Gene A., Burlington, CT 06013 (US); Adams, Leland R., Ansonia, CT 06401 (US); Helfer, Joel N., Cheshire, CT 06410 (US); Backman, Alan B., Milford, CT 06460 (US); Lehman, Adam I., Northford, CT 06472 (US); Fowler, David N., Cheshire, CT 06410 (US)
(74) Representative: Soames, Candida Jane

(57) **Abstract**

The present disclosure relates to devices for introducing a port (10) within a body cavity. The system includes a compressible port (10) that collapses radially for positioning into an introducer (12) that is inserted into an incision. Methods for using the devices are also described.

## Description

### CROSS REFERENCE TO RELATED APPLICATION

The present application claims the benefit of and priority to U.S. Provisional Application Serial No. 61/238,228 filed on August 31, 2009, the entire contents of which are incorporated herein by reference.

### BACKGROUND

### Technical Field

The present disclosure relates generally to a port assembly for use in minimally invasive surgical procedures, such as endoscopic or laparoscopic type procedures, and more particularly to a device and a method for introducing an instrument within a body cavity.

### Background of Related Art

Minimally invasive procedures are continually increasing in number and variation. In particular, certain techniques involve a surgeon operating through a single entry point, typically a patient's navel by providing a device that permits multiple instrument access through a single incision. These procedures are similar to other laparoscopic surgeries in that the patient is under general anesthesia, insufflated, and laparoscopic visualization is utilized. Since the procedure is performed through the navel, patients benefit from less post-operative pain, fewer complications, and better cosmetic results than is achievable from a traditional multi-port laparoscopic procedure.

Once an incision is made in a patient's skin, typically inferior to the patient's umbilicus, the patient is typically prepared for laparoscopic surgery using the Kelly clamp method. The Kelly clamp method involves spreading, separating, and dividing subcutaneous tissue, i.e., dissection. A surgeon's ability to properly place an access device that provides multiple instrument access through a single incision may be complicated due to the limited length of the Kelly's clamp's arm and handle. Furthermore, since the device is held in the palm of a surgeon's hand, sufficient visibility may not be possible without overly dilating the incision thereby compromising the seal. Improper loading of the Kelly clamp may result in the clamp's tips, typically formed from metal, coming into unintentional contact with the surgical area resulting in injury. Removal of the Kelly clamp after installation of the port may also present similar challenges.

### SUMMARY

Disclosed herein are devices and methods for introducing a port within a body cavity.

In an embodiment, a method of introducing a port within a body cavity is disclosed including the steps of placing a compressible port into a membrane, the membrane connected to a substantially rigid rod positioned within a lumen of the compressible port; advancing the port into a body cavity by moving the rod axially through the lumen compressing the compressible port into the body cavity, and once the compressible port is in place, removing the rod through the lumen, thereby removing the membrane and leaving the compressible port within the body cavity. The depth of advancement of the rigid tube within the body cavity may be determined and controlled by utilizing tactile feedback features on the rigid tube. The rod may be adapted to advance incrementally through the introducer. The introducer may include a slot adapted to receive an insufflation valve stem.

In another embodiment, a method of introducing a port within a body cavity is disclosed including the steps of placing a compressible port into a membrane, pulling the membrane into a substantially rigid tube, thereby compressing the compressible port and advancing the compressible port into a body cavity, and removing the membrane from the rigid tube while leaving the port within the body cavity.

In still another embodiment, a method of introducing a port within a body cavity is disclosed including the steps of enclosing a compressible port within a vacuum bag, reducing a pressure within the vacuum bag, thereby compressing the compressible port, pulling the bag through a rigid tube inserted into a body cavity, and removing the vacuum bag while leaving the compressible port within the body cavity.

In a further embodiment, a method of introducing a port within a body cavity is disclosed including the steps of enclosing a compressible port within a vacuum bag, reducing a pressure within the vacuum bag relative to a pressure outside of the bag, thereby compressing the compressible port, pulling the vacuum bag containing the compressible port through a rigid tube inserted into a body cavity, and removing the vacuum bag while leaving the compressible port within the body cavity.

In an embodiment, a system for introducing a port within a body cavity is disclosed including a compressible port, an introducer that is substantially rigid, and a rod positioned within a lumen of the compressible port and attached to a membrane attached to a distal end of the rod, wherein the compressible port is coupled to the membrane and adapted to compress and radially collapse into the introducer upon displacing the rod axially through the introducer.

In another embodiment, a system for introducing a port within a body cavity includes an introducer having a proximal end and a distal end, the proximal end having a wider diameter than the distal end, and a compressible port coupled to a membrane, the membrane adapted to pass through the introducer from the proximal end to the distal end and to pass over an exterior of the introducer upon exiting the distal end. The system may have the proximal end of the introducer configured and dimensioned to be recognizable through tactile feedback, thereby allowing a clinician to position the introducer within a body cavity.

In a further embodiment, a system for introducing a port within a body cavity includes an introducer configured and dimensioned to receive a compressible port housed within a vacuum bag, the vacuum bag adapted to create a pressure differential between an interior of the vacuum bag relative to an exterior of the vacuum bag, thereby compressing the compressible port upon reduction of the pressure in the interior of the vacuum bag. The vacuum bag may be adapted to be removed from the introducer after placement of the compressible port within a body cavity.

In a still further embodiment, a system for introducing a port within a body cavity includes placing a distal end of the compressible port within a heat sensitive membrane such that the distal end is compressed upon the application of heat to the heat sensitive membrane. Compression of the distal end of the compressible port facilitates placement of the compressible port within the incision of the patient. Upon satisfactory placement of the compressible port, the membrane may be removed from the surgical site by opening the membrane along a perforation made along a side of the membrane.

The various aspects of the present disclosure will be more readily understood from the following detailed description when read in conjunction with the appended figures.

### BRIEF DESCRIPTION OF THE DRAWINGS

Embodiments of the disclosure will be described with reference to the accompanying drawings in which:

Fig. 1 is an isometric view of an introducer;

Fig. 2 is an isometric view of a compressible port;

Fig. 3 is an embodiment of a port introducer in accordance with the present disclosure;

Fig. 4A is another embodiment of a port introducer in accordance with the present disclosure shown in a first state;

Fig. 4B is the port introducer of Fig. 4A shown in a second state;

Fig. 5 is yet another embodiment of a port introducer in accordance with the present disclosure;

Fig. 6A is a still further embodiment of a port introducer in accordance with the present disclosure shown in a first state; and

Fig. 6B is the port introducer of Fig. 6A shown in a second state.

### DETAILED DESCRIPTION

Particular embodiments of the present disclosure will be described herein with reference to the accompanying figures. In the following description, well known functions or constructions are not described in detail to avoid obscuring the present disclosures with unnecessary detail. As shown in the figures and as described throughout the following descriptions, and as is traditional when referring to relative positioning on an object, the term "proximal" refers to the end of the device that is closer to the user and the term "distal" refers to the end of the apparatus that is farther from the user.

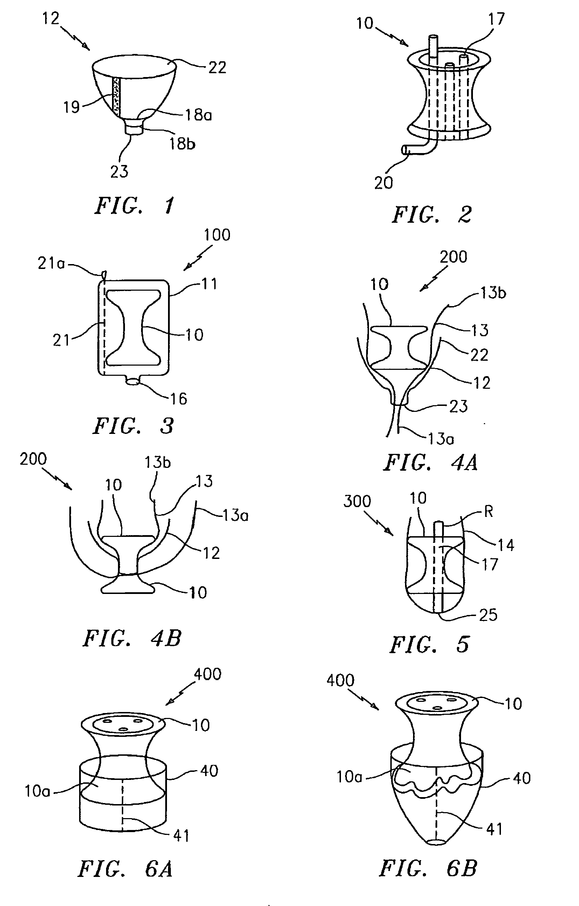

An introducer 12 (Fig. 1) is configured and dimensioned to receive a compressible port 10 (Fig. 2), that is formed from a compliant material and has a plurality of throughholes 17 and optionally an insufflation valve stem 20 within one of the throughholes 17, within the compressible port 10. The introducer 12 has a funnel shape to facilitate the translation of the compressible port 10 from a larger opening 22 to a smaller opening 23. The larger opening 22 may be tapered. A slot 19 may be formed along a side of the introducer 12 to accommodate the insufflation valve stem 20 therethrough. Tactile and/or visual indicators placed on the introducer 12 may assist a clinician in determining the relative position of the compressible port 10 therein. Such indicators may include an indicator line 18a or a bump 18b.

A port introducer 100 and a method of use will now be described with reference to Fig. 3. The port introducer 100 includes a vacuum bag 11 adapted to receive the compressible port 10 therein. The vacuum bag 11 includes a valve 16 and a stress concentration line 21 that can be used to separate the vacuum bag 11 by pulling a tab 21a.

By drawing air out of the vacuum bag 11 through the valve 16, the volume of the vacuum bag is reduced which in turn causes a corresponding reduction in size of the compressible port 10 placed within the vacuum bag 11. Once the volume of the vacuum bag 11 containing the compressible port 10 therein has been reduced in size, the vacuum bag 11 may be placed into an introducer, such as introducer 12 described above, that has been placed within an incision made within a patient. To assist in determining the relative position of the compressible port 10 within the vacuum bag 11 and within the incision, the vacuum bag 11 may be formed from a transparent material. Once the compressible port 10 is positioned as desired, the vacuum bag 11 may be removed from the surgical site by pulling the tab 21a thereby opening the vacuum bag 11 along the stress concentration line 21 while leaving the compressible port 10 within the incision. The compressible port 10 expands to substantially approximate the shape and size of the incision.

Another embodiment of a port introducer will now be described with reference to Figs. 4A and 4B. A port introducer 200 includes a membrane 13 adapted to grasp the compressible port 10 therein. The membrane 13 has a generally tubular shape and is formed from a flexible material and is configured and adapted to be placed in the introducer 12. As shown in Fig. 4A, the compressible port 10 may be stored within the introducer 12 in an uncompressed state prior to use.

Deployment of the compressible port 10 is achieved by placing the introducer 12 containing the membrane 13 having the compressible port 10 placed therein within the incision of the patient. The compressible port 10 may be incrementally advanced through the introducer 12 and into the incision by pulling on a distal portion 13a of the membrane 13, as seen in Fig. 4B.

In yet another embodiment, a port introducer 300 will now be described with reference to Fig. 5. As seen in Fig. 5, a rod R is placed within one of the throughholes 17 of the compressible port 10. A membrane 14 adapted to grip the compressible port 10 therein is coupled to the rod R such that advancement of the rod R will result in translation of the compressible port 10. The membrane 14 may be coupled to the rod R at a distal point 25 of the rod R. The port introducer 300 may be placed within the introducer 12 and advanced through the introducer 12 and into the patient's incision by pushing on the rod R. Once the compressible port 10 is positioned as desired with the incision of the patient, the rod R may be removed through the throughhole 17 thereby removing the rod R along with the membrane 14 from the surgical site.

In a still further embodiment, a port introducer 400 including a membrane 40 will now be described with reference to Figs. 6A and 6B. The membrane 40 has a generally tubular shape and is adapted to shrink in response to the application of heat. The membrane 40 includes a perforation 41 along a side of the membrane 40.

By placing the membrane 40 around a distal portion 10a of the compressible port 10, the distal portion 10a can be compressed through the application of heat to the membrane 40. Upon the application of heat to the membrane 40, the membrane 40 compresses the distal portion 10a to facilitate placement of the compressible port 10 within the incision of the patient, optionally by translating the compressible port 10 through the introducer 12. Once the compressible port 10 is in a desired position within the incision, the membrane 40 can be removed by opening the membrane 40 along the perforation 41 allowing the membrane to be removed from the surgical site. Membrane 40 will reduce in size in the radial and axial dimension.

It will be understood that various modifications may be made to the embodiments disclosed herein. Therefore, the above description should not be construed as limiting, but merely as exemplifications of particular embodiments. Those skilled in the art will envision other modifications within the scope and spirit of the claims appended hereto.

## Claims

1. A system for introducing a port within a body cavity comprising;
a compressible port;
an introducer that is substantially rigid; and
a rod positioned within a lumen of the compressible port and attached to a membrane attached to a distal end of the rod, wherein the compressible port is coupled to the membrane and adapted to compress and radially collapse into the introducer upon displacing the rod axially through the introducer.

2. A system for introducing a port within a body cavity comprises:
an introducer having a proximal end and a distal end, the proximal end having a larger diameter than the distal end; and
a compressible port coupled to a membrane, the membrane adapted to pass through the introducer from the proximal end to the distal end and to pass over an exterior of the introducer upon exiting the distal end.

3. The system of claim 2 further including structure located on the proximal end of the introducer for providing tactile feedback.

4. A system for introducing a port within a body cavity comprising:
an introducer configured and dimensioned to receive a compressible port housed within a vacuum bag, the vacuum bag adapted to create a pressure differential between an interior of the vacuum bag relative to an exterior of the vacuum bag, thereby compressing the compressible port upon reduction of the pressure in the interior of the vacuum bag.

5. The system of claim 4 wherein the vacuum bag is adapted to be removed from the introducer after placement of the compressible port within a body cavity.

6. The system of claim 4 or claim 5, wherein a distal end of the compressible port is positioned inside a heat sensitive membrane such that the distal end is compressed upon the application of heat to the heat sensitive membrane.

7. The system of claim 6 further including a perforation on the heat sensitive membrane that facilitates separation of the compressible port from the heat sensitive membrane.

8. A compressible port of claim 1 or claim 2 for use in a method of inroducing a port into a body cavity wherein the compressible port is placed into a membrane, the membrane connected to a substantially rigid rod positioned within a lumen of the compressible port;
advancing the port into a body cavity by moving the rod axially through the lumen compressing the compressible port into the body cavity; and
removing the rod through the lumen, thereby removing the membrane and leaving the compressible port within the body cavity.

9. The compressible port of claim 8 further comprising utilizing tactile feedback features on the rigid tube for adjusting a depth of advancement of the rigid tube.

10. The compressible port of claim 8 wherein the rod is adapted to advance incrementally through the introducer.

11. The compressible port of claim 10 wherein the introducer includes a slot adapted to receive an insufflation valve stem.

12. A compressible port according to claim 1 or claim 2 for use in a method of introducing a port within a body cavity comprising:
placing a compressible port into a membrane;
pulling the membrane into a substantially rigid tube, thereby compressing the compressible port;
advancing the compressible port into a body cavity; and
removing the membrane from the rigid tube while leaving the port within the body cavity.

13. A compressible port according to claim 1 or claim 2 for use in a method of introducing a port within a body cavity comprising:
enclosing a compressible port within a vacuum bag;
reducing a pressure within the vacuum bag, thereby compressing the compressible port;
pulling the bag through a rigid tube inserted into a body cavity; and
removing the vacuum bag while leaving the compressible port within the body cavity.

14. The compressible port of claim 13 wherein reducing a pressure within the vacuum bag is relative to a pressure outside of the vacuum bag.
